Europäisches Patentamt

European Patent Office

Office européen des brevets

(19)

(11) **EP 0 873 190 B1**

(12) **FASCICULE DE BREVET EUROPEEN**

(45) Date de publication et mention
de la délivrance du brevet:
**08.12.1999 Bulletin 1999/49**

(21) Numéro de dépôt: **97900626.9**

(22) Date de dépôt: **10.01.1997**

(51) Int. Cl.⁶: $B01J\ 23/02$, $B01J\ 23/04$

(86) Numéro de dépôt international:
**PCT/FR97/00045**

(87) Numéro de publication internationale:
**WO 97/25141 (17.07.1997 Gazette 1997/31)**

(54) **NOUVEAU CATALYSEUR D'ALKYLATION, SON PROCEDE DE PREPARATION ET SON UTILISATION DANS DES PROCEDES D'ALKYLATION**

**NEUER ALKKYLIERUNGSKATALYSATOR, IHRES VERFAHREN, HERSTELLUNG UND VERWENDUNG IN ALKYLIERUNGS-VERFAHREN**

**NOVEL ALKYLATION CATALYST, METHOD FOR PREPARING SAME, AND USE THEREOF IN ALKYLATION METHODS**

(84) Etats contractants désignés:
**AT BE CH DE DK ES FI FR GB GR IE IT LI LU NL PT SE**

(30) Priorité: **11.01.1996 FR 9600248**

(43) Date de publication de la demande:
**28.10.1998 Bulletin 1998/44**

(73) Titulaire:
**TOTAL RAFFINAGE DISTRIBUTION S.A.
92800 Puteaux (FR)**

(72) Inventeurs:
- **NASCIMENTO, Pedro
  F-76620 Le Havre (FR)**
- **SZABO, Georges
  F-76290 Montivilliers (FR)**
- **MILAN, Alain
  F-76290 Montivilliers (FR)**

(74) Mandataire: **Jolly, Jean-Pierre
  Cabinet Jolly
  54, rue de Clichy
  75009 Paris (FR)**

(56) Documents cités:
**EP-A- 0 550 331          GB-A- 2 252 104**

**Description**

La présente invention concerne un nouveau catalyseur d'alkylation, son procédé de préparation et son utilisation dans des procédés d'alkylation, notamment dans des réactions d'alkylation de l'isobutane par des oléfines légères.

C'est à cette application particulière du catalyseur conforme à l'invention que l'on se référera plus spécifiquement ci-après, en raison de son importance économique, mais l'invention n'est pas limitée, bien entendu, à cette utilisation.

Il est connu que l'alkylation de l'isobutane par des oléfines légères, dont le nombre d'atomes de carbone est compris entre 3 et 5 (notée ci-après oléfines $C_3$ à $C_5$), permet d'obtenir en particulier un alkylat possédant un indice d'octane élevé et une teneur en composés insaturés ou aromatiques minimale. Ces propriétés intéressantes permettent d'utiliser cet alkylat d'une manière plus importante dans la formulation des essences, ce qui est actuellement recherché par les pétroliers.

Ce type de réaction ne peut être mis en oeuvre que grâce à des catalyseurs acides, voire très acides, et on constate notamment une réduction des réactions parasites telles que les réactions de polymérisation, qui produisent des hydrocarbures insaturés.

Parmi ces catalyseurs, on peut citer les catalyseurs de type acide sulfurique ou acide fluorhydrique, qui sont, dans les procédés d'alkylation, mis en contact sous forme liquide avec le mélange liquide isoparaffine-oléfine pour former une émulsion.

Toutefois, ces procédés posent d'importants problèmes de sécurité, de corrosion et d'environnement.

C'est ainsi que, certaines législations nationales se montrant de plus en plus sévères vis-à-vis de ces problèmes, les spécialistes ont été contraints à se tourner en particulier vers le développement de catalyseurs préparés à partir de solides acides.

Parmi ces catalyseurs acides de type solide, on peut citer les catalyseurs contenant des acides de Lewis et/ou de Bronsted déposés sur divers supports inorganiques, tels que décrits dans les brevets US 3 975 299 A, US 3 852 371 A, US 3 979 476 A et US 4 613 723 A.

On peut également citer les catalyseurs acides de type solide supporté à base d'alumine chlorée, décrits dans les brevets US 3 240 840 A, US 3 523 142 A, US 4 066 716, US 4 083 800 A, US 4 066 716 A, US 4 113 657 A, US 4 138 444 A ou US 2 999 074 A.

On constate, toutefois, que ces catalyseurs présentent une activité insuffisante, une sélectivité pour l'alkylation peu importante et qu'ils ne répondent pas réellement aux besoins de stabilité que l'on recherche généralement pour un catalyseur.

De plus, certains catalyseurs nécessitent des températures de réaction inférieures à la température ambiante, ce qui entraîne des coûts de mise en oeuvre relativement élevés.

En poursuivant ses travaux dans ce domaine, la Demanderesse a établi, de façon surprenante, que le fait d'ajouter au support des catalyseurs acides, de type solide supporté à base d'alumine chlorée, des métaux appartenant aux groupes IA et IIA de la Classification Périodique des éléments, permet d'améliorer les performances des catalyseurs ainsi préparés.

Des buts de la présente invention sont par conséquent :

- d'une part, d'augmenter la sélectivité et/ou la stabilité de ce type de catalyseur, tout en conservant à celui-ci une activité satisfaisante dans son application à l'alkylation de l'isobutane par des oléfines légères ;
- d'autre part, de permettre de réaliser l'alkylation de l'isobutane dans une large gamme de températures, en particulier aux environs de la température ambiante, avec pour conséquence une mise en oeuvre plus aisée.

A cet effet, l'invention a pour premier objet un catalyseur d'alkylation comprenant au moins un support poreux constitué d'alumine, à la surface duquel est présent au moins un halogène, ledit catalyseur étant caractérisé en ce qu'il contient en outre entre 0,005 et 1 % en poids d'au moins un métal appartenant aux groupes IA ou IIA de la Classification Périodique des éléments.

L'alumine jouant le rôle de support pourra présenter une porosité telle que sa surface spécifique déterminée par la méthode B.E.T. est comprise entre 5 et 400 $m^2$/g.

L'halogène présent à la surface du support pourra être du chlore dont la teneur sera comprise entre 2 et 10 % en poids, de préférence entre 3 et 8 % en poids.

La teneur du catalyseur en métal ou en métaux des groupes IA et IIA de la Classification Périodique sera avantageusement comprise entre 0,05 et 0,5 % en poids.

Le catalyseur selon l'invention pourra contenir éventuellement un métal appartenant au groupe VIII de la Classification Périodique des Eléments, de manière à faciliter sa régénération. On pourra citer par exemple le platine.

Le catalyseur selon l'invention pourra être préparé par les moyens usuels de la technique antérieure, bien connus de l'homme du métier.

2

EP 0 873 190 B1

Une des méthodes de préparation pourra consister, dans un premier temps, à déposer le métal ou les métaux appartenant aux groupes IA ou IIA de la Classification Périodique par imprégnation du support en alumine du catalyseur avec une solution aqueuse contenant un sel d'au moins l'un de ces métaux.

Cette solution aqueuse peut être à base de nitrates, de chlorures ou de sulfates, mais, comme il est décrit ci-après, le catalyseur doit subir ultérieurement une chloration, et la Demanderesse préfère donc utiliser, pour l'imprégnation du support, une solution aqueuse de chlorure de l'un des métaux énoncés plus haut.

Parmi ces métaux, la Demanderesse a testé avec succès le lithium, le sodium, le potassium, le césium, le magnésium et le baryum.

La phase d'imprégnation par la solution aqueuse de sel de métal est ensuite suivie d'une évaporation de cette solution dans un évaporateur rotatif.

Après ces deux étapes, le catalyseur est séché à 120°C puis calciné à 500°C dans un four à moufle.

On pourra également effectuer l'imprégnation du support du catalyseur par une méthode dite d'imprégnation à sec, bien connue de la technique antérieure, ou par toute autre méthode d'imprégnation.

Dans un deuxième temps, on effectuera la chloration du support ainsi imprégné, en utilisant par exemple de l'acide chlorhydrique gazeux anhydre sous azote et/ou hydrogène, à des températures pouvant être supérieures à 650°C, ou par toute autre méthode de chloration.

Un second objet de l'invention est par conséquent constitué par un procédé de préparation d'un tel catalyseur, caractérisé par les phases successives suivantes :

- on préimprègne un support poreux constitué d'alumine d'un sel d'au moins un métal appartenant aux groupes IA et IIA de la Classification Périodique des éléments, à sec ou à l'aide d'une solution aqueuse dudit sel ;
- on sèche éventuellement le support ainsi imprégné ;
- on calcine le support ;
- on procède enfin à la chloration dudit support.

Le catalyseur ainsi préparé est alors prêt pour une utilisation dans un procédé d'alkylation et, en particulier, pour l'alkylation de l'isobutane par des oléfines légères, notamment par des butènes.

Ces utilisations du catalyseur constituent par conséquent d'autres objets de l'invention

Dans le cas de l'alkylation de l'isobutane par des oléfines légères, le procédé peut être conduit dans les conditions suivantes:

- température : comprise entre -20 et 100°C, de préférence entre -5 et 35°C,
- pression : suffisante pour maintenir le mélange des réactifs à l'état liquide dans le réacteur,
- vitesse spatiale horaire, exprimée en p.p.h. (poids d'oléfines passant sur le catalyseur par unité de poids de catalyseur et par heure) : comprise entre 0,001 et 10 $h^{-1}$, de préférence entre 0,05 et 3 $h^{-1}$,
- rapport molaire d'isobutane sur oléfines : compris entre 1 et 100.

Le réacteur dans lequel s'effectue la réaction d'alkylation peut être un réacteur mettant en oeuvre le catalyseur en lit fixe, en lit mobile, en lit fluide, ou encore en suspension dans la phase liquide des réactifs soumise à une agitation efficace.

La Demanderesse a ainsi utilisé avec succès un réacteur mettant en oeuvre le catalyseur en lit fixe, qui pourra être rempli d'isobutane préalablement à la mise en route de la réaction d'alkylation, c'est-à-dire avant l'injection du mélange isobutane-oléfine dans le réacteur. Le fait de travailler en excès d'isobutane par rapport à l'oléfine (rapport molaire compris entre 1 et 100) permet de limiter les réactions secondaires.

L'activité du catalyseur selon l'invention est mesurée par la conversion des oléfines.

Cette conversion des oléfines est calculée selon la formule suivante:

$$\text{Conversion} = \frac{\text{débit massique d'oléfines introduites - débit massique d'oléfines en sortie}}{\text{débit massique d'oléfines introduites}}$$

Par ailleurs, on sait que l'alkylat obtenu à partir de l'alkylation de l'isobutane par les oléfines légères contient différents produits tels que, en particulier, des hydrocarbures en $C_5$ à $C_7$, des hydrocarbures plus lourds en $C_8$, dont les triméthylpentanes (notés TMP) et les diméthylhexanes (notés DMH), et enfin des hydrocarbures lourds en $C_{9+}$.

La sélectivité du catalyseur pour l'alkylation est mesurée par :

- la sélectivité en alkylat obtenu, représentée par la sélectivité en composés $C_{5+}$ ;
- la composition de l'alkylat obtenu, représentée par :

3

- la fraction des $C_5$ - $C_7$ dans les $C_{5+}$ ;
- la fraction des $C_8$ dans les $C_{5+}$ ;
- la fraction des $C_{9+}$ dans les $C_{5+}$ ;

selon les formules suivantes :

$$\text{Sélectivité en } C_{5+} = \frac{\text{débit massique des } C_{5+} \text{ poduits}}{\text{débit massique des oléfines introduites - débit massique des oléfines en sortie}}$$

$$\text{Fraction des } C_5 \text{ - } C_7 \text{ dans les } C_{5+} = \frac{\text{débit massique des } [C_5 + C_6 + C_7]}{\text{débit massique des } C_{5+}}$$

$$\text{Fraction des } C_8 \text{ dans les } C_{5+} = \frac{\text{débit massique des } C_8}{\text{débit massique des } C_{5+}}$$

$$\text{Fraction des } C_{9+} \text{ dans les C5+} = \frac{\text{débit massique des } [C_9 + C_{9+}]}{\text{débit massique des } C_{5+}}$$

Lorsque la réaction d'alkylation s'effectue correctement, c'est-à-dire lorsque le catalyseur utilisé est performant, la sélectivité en $C_{5+}$ est voisine de 204 % tout en conservant une conversion des oléfines satisfaisante.

Cette valeur peut être quelque peu inférieure ou supérieure dans le cas où il se produit des réactions secondaires telles que, par exemple, respectivement une réaction d'oligomérisation des oléfines ou une réaction d'autoalkylation de l'isobutane.

De plus, comme le montrent les exemples qui suivent, le catalyseur selon l'invention permet d'obtenir un alkylat contenant une grande quantité de composés $C_8$ et en particulier de composés TMP, dont la fraction dans les $C_8$ est mesurée selon la formule suivante :

$$\frac{\text{TMP}}{C_8} = \frac{\text{débit massique des (2,2,4-TMP + 2,2,3-TMP + 2,3,4-TMP + 2,3,3-TMP)}}{\text{débit massique des } C_8}$$

(les composés TMP sont un mélange des isomères suivants : 2,2,4 - triméthylpentane, 2,2,3 - triméthylpentane 2,3,4 - triméthylpentane et 2,3,3, - triméthylpentane).

Les exemples suivants illustrent la mise en oeuvre de l'invention et présentent ses avantages, sans toutefois en limiter la portée.

EXEMPLE 1

Cet exemple décrit la préparation d'un catalyseur selon l'art antérieur.

Ce catalyseur, noté A, est préparé selon le mode de préparation suivant.

Une quantité de 25 g d'alumine, dont la surface spécifique déterminée par BET est égale à 200 m2/g, est utilisée comme support métallique réfractaire du catalyseur.

Cette alumine est commercialisée par la Société AKZO sous l'appellation CK 300.

Le support est laissé sous azote à 500°C pendant une nuit. Ensuite, il subit une chloration par un mélange gazeux d'acide chlorhydrique et d'hydrogène (proportions 80 /20) à 670°C pendant 2 heures.

Le catalyseur A est donc une alumine chlorée contenant environ 5,5 % en poids de chlore.

EXEMPLE 2

Cet exemple concerne la préparation de catalyseurs selon l'invention.

Les catalyseurs, notés de B à G, sont préparés selon le mode de préparation suivant.

Le support métallique réfractaire des catalyseurs est l'alumine utilisée dans l'exemple 1.

On procède à l'imprégnation de 50 g de ce support par mise en contact avec 125 cm$^3$ d'une solution aqueuse de chlorures d'un métal du groupe suivant : lithium, sodium, potassium, césium, magnésium ou baryum.

Les catalyseurs B, C, D, E, F et G sont préparés respectivement à partir de lithium, sodium, potassium, césium, magnésium et baryum. Différentes concentrations de chacun de ces métaux ont été étudiées.

La solution aqueuse est ensuite évaporée dans un évaporateur rotatif et les catalyseurs ainsi obtenus sont séchés à 120°C, puis calcinés à 500°C, pendant 2 heures dans un four à moufle.

La chloration des catalyseurs imprégnés de métal est effectuée selon la méthode décrite dans l'Exemple 1.

Pour chacun des catalyseurs ainsi préparés, le support contient entre 5 et 6 % en poids de chlore et des quantités variables de métal, comme le montre le Tableau I ci-après, dans l'exemple 3.

EXEMPLE 3

Cet exemple vise à comparer les performances du catalyseur A de l'art antérieur et les catalyseurs B à G de l'invention, préparés selon les Exemples 1 et 2.

Ces deux types de catalyseurs sont testés dans la réaction d'alkylation de l'isobutane par les butènes dans les conditions suivantes :

- charge : isobutane + butène-2-trans, avec un rapport molaire isobutane sur butène-2-trans voisin de 14,
- masse de chaque catalyseur : 8 g,
- température de réaction : 0° C,
- vitesse spatiale horaire exprimée en pph (poids d'oléfines passées sur le catalyseur par unité de poids de catalyseur et par heure) : 0,25 h$^{-1}$,
- pression totale dans le réacteur : 30.10$^5$ Pa.

Préalablement à la mise en route de la réaction d'alkylation, on remplit le réacteur d'azote sous la pression de réaction, avant l'injection du mélange isobutane + butène-2-trans.

Pour chacun des catalyseurs A à G, on calcule, après la réaction d'alkylation, le taux de conversion des butènes, la sélectivité en composés $C_{5+}$ obtenus, la fraction des composés $C_5$ à $C_7$, $C_8$, $C_{9+}$ obtenus dans l'alkylat, ainsi que la fraction des composés TMP (triméthylpentanes) obtenus dans les composés $C_8$, selon les formules suivantes :

$$\text{Conversion} = \frac{\text{débit massique des butènes introduits - débit massique des butènes en sortie}}{\text{débit massique des butènes introduits}}$$

$$\text{Sélectivité en C}_{5+} = \frac{\text{débit massique des C}_{5+}\text{ produits}}{\text{débit massique des oléfines introduites - débit massique des oléfines en sortie}}$$

$$\text{Fraction des C}_5\text{ - C}_7\text{ dans les C}_{5+} = \frac{\text{débit massique des [C}_5 + C_6 + C_7]}{\text{débit massique des C}_{5+}}$$

$$\text{Fraction des C}_8\text{ dans les C}_{5+} = \frac{\text{débit massique des C}_8}{\text{débit massique des C}_{5+}}$$

$$\text{Fraction des C}_{9+}\text{ dans les C}_{5+} = \frac{\text{débit massique des [C}_{9+} + C_{9+}]}{\text{débit massique des C}_{5+}}$$

$$\frac{\text{TMP}}{\text{C}_8} = \frac{\text{débit massique des (2,2,4-TMP + 2,2,3-TMP + 2,3,4-TMP + 2,3,3-TMP)}}{\text{débit massique des C}_8}$$

Le Tableau I ci-après rassemble ces résultats.

## Tableau I

| Catalyseurs | A | B | | | C | | D | | E | | F | G |
|---|---|---|---|---|---|---|---|---|---|---|---|---|
| Durée d'utilisation 5 minutes | Sans ajout | % en poids de Li | | | % en poids de Na | | % en poids de K | | % en poids de CS | | % en poids de Mg | % en poids de Ba |
| | | 0,05 | 0,25 | 0,50 | 0,05 | 0,25 | 0,20 | 0,40 | 0,2 | 0,40 | 0,60 | 0,5 |
| Conversion (%) | 93,4 | 96,0 | 98,6 | 89,4 | 91,9 | 95,2 | 87,0 | 77,3 | 86,8 | 84,6 | 83,88 | 90,4 |
| Sélectivité $C_5+$ (%) | 238 | 231 | 226 | 212 | 256 | 212 | 216 | 203 | 243 | 229 | 186 | 237 |
| Composition de l'alkylat (%) | | | | | | | | | | | | |
| $C_5 - C_7$ | 50,6 | 47,3 | 19,3 | 9,2 | 46,8 | 30,7 | 30,4 | 20,4 | 45,0 | 35,9 | 18,6 | 42,5 |
| $C_8$ | 39,8 | 43,1 | 74,8 | 85,7 | 43,5 | 60,9 | 60,3 | 70,7 | 43,1 | 53,4 | 74,2 | 46,9 |
| $C_9'$ | 9,6 | 9,5 | 5,9 | 5,1 | 9,8 | 8,2 | 9,3 | 8,8 | 11,8 | 10,8 | 7,1 | 10,7 |
| $TMP/C_8$ (%) | 69 | 73 | 94 | 97 | 74 | 86 | 84 | 90 | 71 | 77 | 91 | 75 |

Ces résultats montrent que les catalyseurs B à G selon l'invention conduisent à une alkylation de meilleure qualité, puisque les composés lourds $C_8$, en particulier les composés TMP, sont produits en plus grande quantité, tandis que la conversion des butènes reste satisfaisante.

6

EXEMPLE 4

Cet exemple vise à comparer les performances du catalyseur B selon l'invention et du catalyseur A selon l'art antérieur au cours du temps.

La réaction d'alkylation est réalisée dans les mêmes conditions que celles décrites dans l'Exemple 3.

Le Tableau II ci-après rassemble les résultats obtenus pour la conversion des butènes et la sélectivité en $C_{5+}$ mesurées comme décrit dans l'Exemple 3.

Tableau II

| Temps | Catalyseur A | | 0,05% en poids de Li | | 0,15% en poids de Li | | 0,25% en poids de Li | | 0,50% en poids de Li | |
|---|---|---|---|---|---|---|---|---|---|---|
| | Conversion (%) | Sélectivité (%) | Conversion (%) | Sélectivité (%) | Conversion (%) | Sélectivité (%) | Conversion (%) | Sélectivité (%) | Conversion (%) | Sélectivité (%) |
| 5 mn | 93,4 | 238 | 96,0 | 231 | 97,2 | 232 | 99,1 | 212 | 89,4 | 212 |
| 2,17h | 13,7 | 128 | 50,1 | 210 | 63,0 | 223 | 69,2 | 216 | 45,7 | 226 |
| 3,25h | --- | --- | --- | --- | --- | --- | 29,6 | 175 | --- | --- |

Catalyseur B

Ces résultats montrent que le catalyseur B selon l'invention présente une stabilité très nettement supérieure à celle du catalyseur A de l'art antérieur.

EXEMPLE 5

Cet exemple vise à comparer les performances du catalyseur C selon l'invention et du catalyseur A de l'art antérieur selon la température de réaction.

La réaction d'alkylation est réalisée dans les mêmes conditions que celles décrites dans les Exemples 3 et 4, la température étant bien sûr variable et la pression suffisante pour maintenir le mélange des réactifs à l'état liquide dans le réacteur.

Toutefois, le réacteur est cette fois préalablement rempli d'isobutane liquide.

Le Tableau III ci-après rassemble les résultats obtenus pour la conversion des butènes et la sélectivité en $C_{5+}$ mesurées comme décrit dans l'Exemple 3.

Tableau III

| Durée d'utilisation : 2h45 mn | | | | |
|---|---|---|---|---|
| T(°C) | Catalyseur A | | Catalyseur C (0,05% en poids de Na) | |
| | Conversion (%) | Sélectivité $C_{5+}$ (%) | Conversion(%) | Sélectivité $C_{5+}$ (%) |
| - 30 | --- | --- | 30,1 | 192 |
| - 26 | 35,4 | 183 | --- | --- |
| - 20 | --- | --- | 50,4 | 196 |
| - 10 | 44,4 | 184 | 60,2 | 187 |
| 0 | 53,0 | 175 | 70,6 | 179 |
| 24 | 6,9 | 152 | 84,6 | 164 |
| 51 | --- | --- | 77,6 | 156 |

Ces résultats montrent que le catalyseur C selon l'invention permet une meilleure conversion et une meilleure sélectivité en $C_{5+}$ quelle que soit la température de réaction, et permet surtout de travailler à des températures voisines de la température ambiante.

**Revendications**

1. Catalyseur d'alkylation comprenant au moins un support poreux constitué d'alumine à la surface duquel est présent au moins un halogène , le dit catalyseur étant caractérisé en ce qu'il contient en outre entre 0,005 et 1 % en poids d'au moins un métal appartenant aux groupes IA et IIA de la Classification Périodique des éléments.

2. Catalyseur selon la revendication 1, caractérisé en ce que l'halogène est le chlore.

3. Catalyseur selon l'une des revendications 1 et 2, caractérisé en ce qu'il contient entre 2 et 10 % en poids de chlore, de préférence entre 3 et 8 % en poids.

4. Catalyseur selon l'une des revendications 1 à 3, caractérisé en ce qu'il contient entre 0,05 et 0,5 % en poids d'au moins un métal appartenant aux groupes IA et IIA de la Classification Périodique des éléments.

5. Catalyseur selon l'une des revendications 1 à 4, caractérisé en ce que le métal est choisi dans le groupe constitué par le lithium, le sodium, le potassium, le césium, le magnésium et le baryum.

6. Procédé de préparation d'un catalyseur selon l'une des revendications 1 à 5, caractérisé par les phases successives suivantes :

   - on préimprègne un support poreux constitué d'alumine d'un sel d'au moins un métal appartenant aux groupes IA et IIA de la Classification Périodique des éléments, à sec ou à l'aide d'une solution aqueuse dudit sel ;
   - on sèche éventuellement le support ainsi imprégné ;
   - on calcine le support ;

- on procède enfin à la chloration dudit support.

7. Utilisation d'un catalyseur selon l'une des revendication 1 à 5 dans un procédé d'alkylation.

8. Procédé d'alkylation de l'isobutane par des oléfines légères mettant en oeuvre le catalyseur selon l'une des revendications 1 à 5, caractérisé en ce que l'alkylation est réalisée à une température comprise entre -20 et 100°C, de préférence entre -5 et 35°C, sous une pression suffisante pour maintenir le mélange des réactifs à l'état liquide, avec une vitesse spatiale horaire, exprimée en poids d'oléfine passant sur le catalyseur par unité de poids du catalyseur et par heure, comprise entre 0,001 et 10 $h^{-1}$, de préférence entre 0,05 et 3 $h^{-1}$, et avec un rapport molaire isobutane sur oléfines compris entre 1 et 100.

9. Procédé selon la revendication 8, caractérisé en ce que les oléfines légères sont des butènes.

**Claims**

1. An alkylation catalyst comprising at least one porous support consisting of alumina, at the surface of which at least one halogen is present, said catalyst being characterised in that it additionally contains between 0.005 and 1 wt.% of at least one metal belonging to groups IA and IIA of the periodic system of elements.

2. A catalyst according to claim 1, characterised in that the halogen is chlorine.

3. A catalyst according to one of claims 1 and 2, characterised in that it contains between 2 and 10 wt.% of chlorine, preferably between 3 and 8 wt.%.

4. A catalyst according to one of claims 1 to 3, characterised in that it contains between 0.05 and 0.5 wt.% of at least one metal belonging to groups IA and IIA of the periodic system of elements.

5. A catalyst according to one of claims 1 to 4, characterised in that the metal is selected from the group comprising lithium, sodium, potassium, caesium, magnesium and barium.

6. A process for the production of a catalyst according to one of claims 1 to 5, characterised by the following successive phases:

   - a porous support consisting of alumina is preimpregnated with a salt of at least one metal belonging to groups IA and IIA of the periodic system of elements, either dry or by means of an agueous solution of said salt;
   - the support impregnated in this manner is optionally dried;
   - the support is calcined;
   - said support is finally chlorinated.

7. Use of a catalyst according to one of claims 1 to 5 in an alkylation process.

8. Process for alkylating isobutane with light olefins using the catalyst according to one of claims 1 to 5, characterised in that alkylation is performed at a temperature between -20 and 100°C, preferably between -5 and 35°C, under a pressure sufficient to maintain the mixture of reagents in the liquid state, at an hourly space velocity, stated as the weight of olefin passing over the catalyst per unit weight of catalyst per hour, of between 0.001 and 10 $h^{-1}$, preferably between 0.05 and 3 $h^{-1}$, and with a molar ratio of isobutane to olefins of between 1 and 100.

9. Process according to claim 8, characterised in that the light olefins are butenes.

**Patentansprüche**

1. Alkylierungskatalysator, enthaltend mindestens einen porösen Träger aus Aluminiumoxid, auf dessen Oberfläche mindestens ein Halogen vorhanden ist, dadurch gekennzeichnet, daß der Katalysator auch zwischen 0,005 und 1 Gew.-% mindestens eines Metalls, welches den Gruppen I A und II A des Periodensystems der Elemente angehört, enthält.

2. Katalysator nach Anspruch 1, dadurch gekennzeichnet, daß das Halogen Chlor darstellt.

3. Katalysator nach einem der Ansprüche 1 und 2, dadurch gekennzeichnet, daß er zwischen 2 und 10 Gew.-%, vorzugsweise zwischen 3 und 8 Gew.-% Chlor, enthält.

4. Katalysator nach einem der Ansprüche 1 bis 3, dadurch gekennzeichnet, daß er zwischen 0,05 und 0,5 Gew.-% mindestens eines Metalls, das den Gruppen I A und II A des Periodensystems der Elemente angehört, enthält.

5. Katalysator nach einem der Ansprüche 1 bis 4, dadurch gekennzeichnet, daß das Metall aus der Gruppe, bestehend aus Lithium, Natrium, Kalium, Cäsium, Magnesium und Barium, ausgewählt ist.

6. Verfahren zur Herstellung eines Katalysators nach einem der Ansprüche 1 bis 5, gekennzeichnet durch folgende aufeinanderfolgende Schritte:

   - man vorimprägniert einen porösen Träger aus Aluminiumoxid mit einem Salz mindestens eines Metalls aus den Gruppen I A und II A des Periodensystems der Elemente, auf trockenem Wege oder mit Hilfe einer wäßrigen Lösung dieses Salzes;

   - man trocknet gegebenenfalls den so imprägnierten Träger;

   - man calciniert den Träger;

   - man nimmt schließlich eine Chlorierung dieses Trägers vor.

7. Verwendung eines Katalysators nach einem der Asprüche 1 bis 5 für ein Alkylierungsverfahren.

8. Verfahren zur Alkylierung von Isobutan mit leichten Olefinen unter Verwendung des Katalysators nach einem der Ansprüche 1 bis 5, dadurch gekennzeichnet, daß die Alkylierung bei einer Temperatur zwischen -20°C und 100°C, vorzugsweise zwischen - 5°C und 35°C, unter einem ausreichenden Druck, um das Reaktionsgemisch im flüssigen Zustand zu halten, mit einer stündlichen Raumgeschwindigkeit, ausgedrückt als Gewichtsteile des Olefins, das über den Katalysator geleitet wird, je Gewichtseinheit des Katalysators und Stunde, zwischen 0,001 und 10 $h^{-1}$, vorzugsweise zwischen 0,05 und 3 $h^{-1}$, und mit einem Molverhältnis Isobutan/Olefinen zwischen 1 und 100, durchgeführt wird.

9. Verfahren nach Anspruch 8, dadurch gekennzeichnet, daß man als leichte Olefine Butene verwendet.